(19) Europäisches Patentamt
European Patent Office
Office européen des brevets

(11) Publication number: 0 052 588
A1

# (12) EUROPEAN PATENT APPLICATION

(21) Application number: 81850205.6

(22) Date of filing: 04.11.81

(51) Int. Cl.³: C 07 D 213/38
A 61 K 31/44
//C07D213/48, C07D213/30

(30) Priority: 14.11.80 SE 8007991

(43) Date of publication of application: 26.05.82 Bulletin 82/21

(84) Designated Contracting States: AT CH IT LI NL SE

(71) Applicant: Astra Läkemedel Aktiebolag
Strängnäsvägen 44
S-151 85 Södertälje(SE)

(72) Inventor: Högberg, Thomas
Vallmostigen 5
S-153 00 Järna(SE)

(72) Inventor: Ulff, Carl Bengt Johan
Agärdevägen 9
S-151 47 Södertälje(SE)

(74) Representative: Wurm, Bengt Runio et al,
Patent and Trade Mark Department Ab Astra
S-151 85 Södertälje(SE)

(54) **A novel process for preparing bromophenyl-pyridyl-allylamines.**

(57) A novel process for preparing compounds of the formula

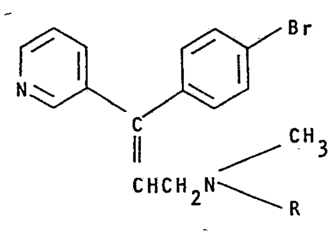

wherein R is H or $CH_3$, comprising reductive amination of an aldehyde of the formula

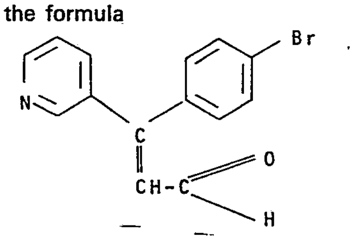

with methylamine or dimethylamine in the presence of a reducing agent, and use of the aldehyde as starting material or intermediate. The end compounds are useful for therapeutic treatment of depressive conditions.

EP 0 052 588 A1

# A novel process for preparing bromophenyl-pyridyl-allylamines

## DESCRIPTION

### Technical field

The present invention relates to a novel process for preparing compounds having therapeutic activity. The invention is further related to the use of a compound as starting material or intermediate in preparation of a therapeutically active compound.

Compounds of the formula

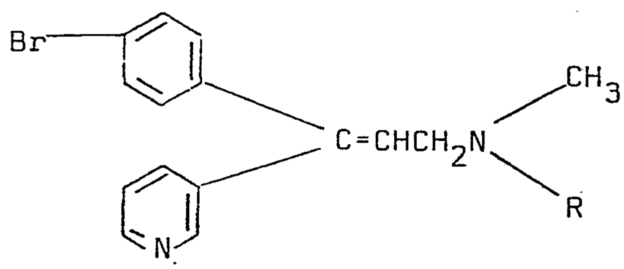

I

wherein R represents hydrogen or methyl, having valuable therapeutic properties especially as antidepressants are known from Belgian Patent Specifications No. 781.105 and No. 835.802. Further processes for preparing such compounds are known from the following Swedish patent applications: No. 7605779-3, No. 7707707-1 and No. 7707708-9.

### Disclosure of invention

### General outline

A main object of the present invention is to provide a novel process for preparing compounds of formula I above as well as therapeutically acceptable salts thereof, said process

having advantages over known processes. A further object is to provide a process for preparation of a compound of formula I in a desired diastereomeric form, and/or permitting a stereospecific synthesis of a compound of formula I. Another object is to provide a convenient process for conversion between compounds of formula I in which R has different meaning or to enable the introduction or removal of a labelled secondary or tertiary amino function in a compound of formula I. Further objects of the invention will be evident from the following description.

Due to the lack of free rotation in the double bond the compounds of formula I exist in two stereoisomeric forms, that is in cis-trans isomers or, according to the IUPAC nomenclature (J. Org. Chem. 35, 2849-2867, September 1970), in an E-form and a Z-form. The compound may be used therapeutically as a mixture of geometrical isomers or in pure E or Z form. For therapeutic purposes the Z isomers are preferred.

The compounds of formula I may be prepared for administration in the form of free bases or their salts with non-toxic acids. Some typical examples of these salts are the hydrobromide, hydrochloride, phosphate, sulphate, citrate, tartrate, malate, maleate and oxalate.

The novel process of the present invention is characterized in making a reductive amination of an aldehyde of the formula

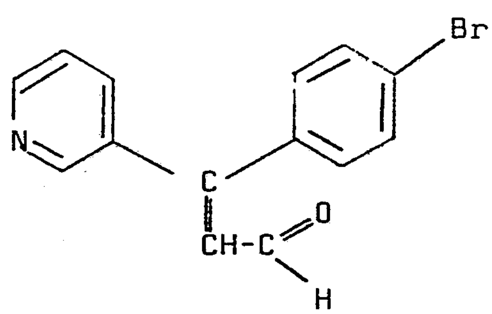

II

with methylamine or dimethylamine in the presence of a reducing agent, to the formation of a compound of formula I. The reducing agent can be e.g. Raney nickel, sodium cyanoborohydride, lithium cyanoborohydride, boranes, sodium borohydride, formic acid, formates, formamides or alcoholic sodium, alcoholic sodium amalgam, alcoholic metal hydrides or alcoholic potassium hydroxide. Alternatively, the reduction may be accomplished electrolytically with metal cathodes like platinum, lead or copper or with a metal/acid system having a metal such as zinc.

When formic acid is used as reducing agent, initial formation of a formate with the reagent methylamine or dimethylamine is suitably done.

Preferably the process of the invention is carried out under non-oxygen gas atmosphere at a temperature between -20$^{o}$ and reflux temperature of the solvent used.

An important advantage of the process of the invention is providing possibilities of preparing the compound of formula I in a desired diastereomeric form. Thus a diastereomerically pure aldehyde may be aminated in a stereospecific way to the pure or substantially pure isomer of the amine of equal configuration. Preferably such stereospecific reduction is carried out with lithium cyanoborohydride or sodium cyanoborohydride in an alcoholic solution at a pH between 4 and 8 at 0-50$^{o}$C, most preferably at room temperature, with an excess of amine. Further, the starting material of formula II may be employed in any desired ratio of diastereomers to the obtention of an end compound of formula I with the same diastereomer ratio. Alternatively, a Z/E mixture or a diastereomer of the aldehyde can be reductively aminated in a non-specific way, e.g. using formic acid, a formate or formamide as reducing agent, giving an isomeric mixture of the amine from which

subsequently any desired isomer can be isolated. Such conditions can be especially useful when isomer conversion is desired. Thus, from a mother lye containing the E-form of a compound of formula I an isomeric mixture of said compound may be prepared via the aldehyde, and the Z-form may be isolated.

An unexpected advantage of the invention is that the aldehyde of formula II is a stable and storable intermediate. This fact renders the process especially useful for production in a commercial scale. A further related advantage is that the aldehyde of formula II is preparable from a corresponding primary, secondary or tertiary allylamine as will be described below. This provides a practically advantageous process for conversion between different allylamines, one or both of which may satisfy formula I above. Interconversion between different compounds of formula I can thus conveniently be made, and further the amino funciton may be replaced with a labelled amino function, such as a tritium labelled amine, or such labelled amino function may be removed. In this respect the invention provides an efficient tool for pharmacological research.

Starting materials

Preparation of the starting materials for the process of the invention according to the formula II may be carried out as outlined below. Thus the starting material may be prepared by oxidation of a compound of formula

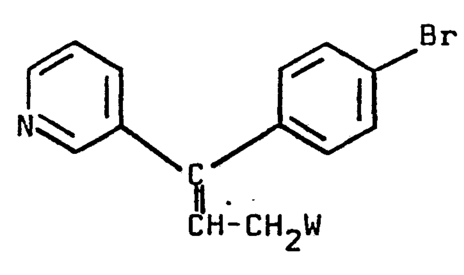

III

wherein W is OH or a leaving group Y, NR'R", NR'H, $NH_2$ or NR'R" ↓ O, wherein R' and R" are alkyl groups having 1-4 carbon atoms with reagents such as manganese dioxide, dimethyl sulfoxide, silver(I), silver(II), iron(III), chromium trioxide reagents, aluminium alkoxides, nickel peroxide, lead tetraacetate and 2,3-dichloro-5,6-dicyano-benzoquinone or by oxidation in one step from a compound of the formula

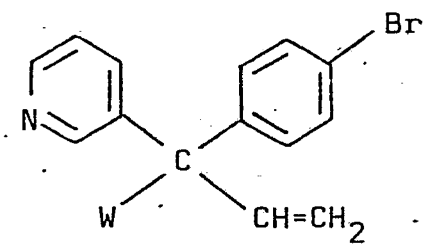

IV

wherein W is as defined above and preferable is OH or a leaving group Y, with an appropriate reagent mentioned above, such as chromic acid/sulfuric acid.

With appropriate choice of NR'R" or NR'H the compounds of formula III also satisfy formula I. Thereby a conversion between different compounds of formula I is made possible, especially a stereospecific such conversion.

The intermediate alcohols of formula III and IV may be prepared by methods known in the art.

A carboxylic acid may be prepared by further oxidation of the aldehyde mentioned above. Such carboxylic acid can be used for reductive amination to a compound of formula I.

## Working Examples

### Preparation of starting materials

Example A (E)-3(4-Bromophenyl)-3-(3-pyridyl-2-propenal

A mixture of 2.0 g (6.3 mmol) (E)-3-(4-bromophenyl)-N,N-dimethyl-3-(3-pyridyl)allylamine (UV Table 1) and 10 g

manganese dioxide (Merck, gefällt) in 40 ml chloroform was stirred at reflux under nitrogen. Two additional portions of 5 g manganese dioxide each were added after 2 h and 5 h, respectively. After totally 7 h all starting amine was consumed and the mixture was filtered. Evaporation gave 1.83 g of a residual yellow oil, which was used directly in the following reductive methylamination.

A portion of the crude aldehyde (0.5 g) was further purified by flash chromatography on 40 g $SiO_2$ (0.040-0.063 mm) with ethyl acetate as eluent. Evaporation of the solvent gave the pure title compound (0.41g)as an oil which solidified on standing. Recrystallization from diisopropyl ether gave 0.22 g off-white needles. M.p. 93-94$^{o}$C.

$^{1}$H NMR (60 MHz, $CDCl_3$): $\delta$ 6.65 (d,1,J=8 Hz, vinyl), 7.25 (AA,2, 2,6-phenyl), 7.70 (BB',2, 3,5-phenyl), 7.4-7.8 (m,2, 4,5-pyridyl), 8.65 (m,1, 2-pyridyl), 8.70 (dd,1, 6-pyridyl), 9.60 (d, J=8 Hz, CHO). MS(70 eV): m/e (rel.-int.): 289/287 (41/42),288/286(34/29), 260/258(13/12), 208(100), 180(46), 179(36), 153(9.2), 152(31), 151(17), 126(10) and 103(31). UV (Table 1).

Example B (Z)-3-(4-Bromophenyl)-3-(3-pyridyl)-2-propenal

(Z)-3-(4-Bromophenyl)-N,N-dimethyl-3-(3-pyridyl)allylamine (base, 1.0 g, 3.1 mmol) and 10 g manganese dioxide (Merck, gefällt) were stirred in 30 ml chloroform at reflux for 4 h. TLC showed minor residual amounts of the starting compound and additional 3 g manganese dioxide (Merck, gefällt) were added. After 30 min the starting compound was consumed and the mixture was filtered. Evaporation gave 0.9 g of a yellow oil, which after recrystallization from diisopropyl ether gave 0.4 g (44%) of pure aldehyde. M.p. 99-100$^{o}$C. NMR, MS and UV spectra were in accordance with Example D below.

Example C (Z)-3-(4-Bromophenyl)-3-(3-pyridyl)-2-propen-1-ol

1-(4-Bromophenyl)-1-(3-pyridyl)-2-propen-1-ol (100 g, 0.345 mol), was heated under reflux for 3 h in 400 ml water and 30 ml conc. sulfuric acid. The reaction mixture was cooled, made alkaline with 45% NaOH and extracted with dichloro - methane. The solvent was evaporated and the residue recrystallized from toluene/hexane and treated with charcoal. Further fractional recrystallizations from toluene gave 29.6 g (30%) of pure Z-isomer. M.p. 131-131.5°.

$^1$H NMR (60 MHz, $CDCl_3$): $\delta$ 3.4 (s, 1, OH), 4.2 (d, 2, allyl), 6.4 (t, 1, vinyl), 7.1 (AA', 2, 2,6-phenyl), 7.4 (BB', 2, 3,5-phenyl), 7.2-7.6 (m, 2, 4,5-pyridyl), 8.4 (m, 1, 2-pyridyl) 8.6 (dd, 1, 6-pyridyl).

MS (70 eV): m/e (rel.int.): 291/289 (M, 15/16), 290/288 (5/4), 273/271 (M-$H_2O$, 9/8), 262/260 (6/7), 248/246 (4/6), 210 (M-Br, 100), 195 (11), 193 (10), 192 (M-$H_2O$-Br, 18), 167 (31), 166 (15), 152 (9) and 104 (21).

UV (Table 1).

Example D (Z)-3-(4-Bromophenyl)-3-(3-pyridyl)-2-propenal

(Z)-3-(4-Bromophenyl)-3-(3-pyridyl)-2-propen-1-ol (10 g, 0.034 mol) was dissolved in 150 ml chloroform and stirred with 30 g manganese dioxide (Merck, gefällt) at room temperature under nitrogen atmosphere for 3 h. The mixture was filtered and the solvent was evaporated to give 10 g crystalline aldehyde, which was recrystallized twice from diisopropyl ether (8.5 g, 86%). M.p. 99-99.5°.

$^1$H NMR (60 MHz, $CDCl_3$): $\delta$ 6.7 (d, 1, J=8 Hz, vinyl), 7.2 (AA', 2, 2,6-phenyl), 7.5 (BB', 2, 3,5-phenyl), 7.4-7.8 (m, 2, 4,5-pyridyl), 8.6 (m, 1, 2-pyridyl), 8.8 (dd, 1, 6-pyridyl), 9.6 (d, 1, J=8 Hz, CHO).

MS (70 eV): m/e (rel.int.): 289/287 (M, 39/41), 288/286 (33/27), 260/258 (M-CHO, 13/13), 208 (M-Br, 100), 180 (42), 179 (34), 153 (28), 152 (16), 126 (10) and 103 (28).

UV (Table 1).

Preparation of end compounds

Example 1 (E)-3-(4-Bromophenyl)-N-methyl-3-(3-pyridyl) allylamine oxalate

To 0.43 g (1.5 mmol) crude (E)-3-(4-bromphenyl)-3-(3-pyridyl)--2-propenal was added 0.91 g (13.5 mmol) methylamine hydrochloride, 10 ml methanol, 0.36 g (9 mmol) sodium hydroxide, 0.13 g (2 mmol) sodium cyanoborohydride and finally 5 g molecular sieves (3 Å). The mixture was stirred at room temperature under nitrogen for 3 days and then 100 ml methanol were added. After filtration the residue was washed with methanol and 2M hydrochloric acid. The methanolic solution was evaporated and the residue dissolved in an aqueous solution at pH 4.5 and washed with ether twice. The aqueous solution was made alkaline, extracted twice with ether and dried over magnesium sulfate. Evaporation gave 0.15 g (33 % overall yield) of the title compound as an oil, which was precipitated in the form of oxalate from hot ethanol (0.11g, 19% overall yield from corresponding tertiary amine). M.p. 192-194$^{o}$C.

MS and NMR were in accordance with an authentic sample.
UV (Table 1)

Example 2 (Z)-3-(4-Bromophenyl)-N,N-dimethyl-3-(3-pyridyl)-allylamine dihydrochloride monohydrate

To 0.58 g (2 mmol) (Z)-3-(4-bromophenyl)-3-(3-pyridyl)-2-propenal was added 1.47 g (18 mmol) dimethylamine hydrochloride, 20 ml methanol, 0.48 g (12 mmol) sodium hydroxide, 0.19 g (3 mmol) sodium cyanoborohydride and finally 5 g molecular sieves (3Å). The mixture (pH 9.6) was stirred at room temperature under nitrogen for 3 days and then 100 ml methanol were added. After filtration the residue was washed with methanol and 2M hydrochloric acid. The methanolic

solution was evaporated and the residue dissolved in an aqueous solution at pH 4.9 and washed with ether twice. The aqueous solution was made alkaline, extracted twice with ether and dried over magnesium sulfate. Evaporation gave 0.48 g (76%) of pure base having a Z/E ratio of 52/48, which was dissolved in hot isopropanol. After addition of 0.36 ml conc. hydrochloric acid the title compound slowly precipitated to give 0.28 g (34%) white crystals. M.p. 191-194$^{o}$C. MS and NMR were in accordance with an authentic sample. UV (Table 1)

<u>Example 3</u> (<u>Z</u>)-3-(4-Bromophenyl)-<u>N</u>,<u>N</u>-dimethyl-3-(3-pyridyl)-allylamine dihydrochloride monohydrate

To 144 mg (0.5 mmol) (<u>Z</u>)-3-(4-bromophenyl)-3-(3-pyridyl)-2 propenal was added 408 mg (5 mmol) $(CH_3)_2NH \cdot HCl$, 5 ml methanol, 63 mg (1 mmol) $NaBH_3CN$ and finally 2 g molecular sieves (3Å). The mixture having pH 5.4 was stirred at room temperature under $N_2$ for 2 days and then 20 ml methanol were added. Work up according to Example 2 gave 88 mg (55%) of pure base containing only 1% E-isomer. Addition of hydrochloric acid gave 70 mg (34%) dihydrochloride monohydrate.

<u>Example 4</u> (Z)-3-(4-Bromophenyl)-<u>N</u>-methyl-3-(3-pyridyl) allylamine oxalate

To 0.58 g (2 mmol) (<u>Z</u>)-3-(4-bromophenyl)-3-(3-pyridyl)-2-propenal were added 0.68 g (10 mmol) methylamine hydrochloride, 10 ml methanol, 0.32 g (8 mmol) sodium hydroxide, 0.19 g (3 mmol) sodium cyanoborohydride and finally 5 g molecular sieves (3Å). The mixture was stirred at room temperature under nitrogen for 3 days and then 100 ml 2M hydrochloric acid were added. After filtration the aqueous solution was made alkaline, extracted twice with ether and dried over magnesium sulfate. Evaporation gave 0.32 g (1.1 mmol) of an oil, which was dissolved in hot ethanol. Oxalic

acid (0.9 mmol, 0.11 g) was added and the title compound precipitated from the solution to give 0.20 g (26%) of a white crystalline substance. M.p. 210-211°C. MS and NMR were in accordance with an authentic sample. UV (Table 1)

<u>Example 5</u> 3-(4-Bromophenyl)-N,N-dimethyl-3-(3-pyridyl)-allylamine

Z-3-(4-Bromophenyl)-3-(3-pyridyl)propenal (288 mg, 1.00 mmol), dissolved in dimethylformamide (1.1 ml), was added to a mixture of formic acid (0.11 ml, 2.4 mmol) and dimethylamine (0.94 ml, 14.6 mmol) at -20°C. The mixture was heated under reflux at 160°C for 3 hours, cooled, diluted with water and extracted with ether at pH 4.0. The aqueous phase was made alkaline and extracted with ether. The organic phase was dried over sodium sulphate and evaporated to give an oil (280 mg, 83% yield). NMR and GLC show the Z/E ratio to be about 45/55, which shows that complete isomerization has taken place.

<u>Example 6</u> Z-3-(4-Bromophenyl-N,N-dimethyl-3-(3-pyridyl)-allylamine

To Z-3-(4-bromophenyl)-3-(3-pyridyl)propenal (288 mg, 1,0 mmol) in 10 ml methanol were added dimethylamine hydrochloride (815 mg, 10 mmol), sodium cyanoborohydride (94 mg, 1.5 mmol) and 2 g molecular sieves (3Å). The mixture (pH 5.8) was stirred for 3 hours at room temperature under $N_2$ and filtered. The filtrate was evaporated, the residue dissolved in 10 ml 2M hydrochloric acid and pH adjusted to 4. After extraction with ether, the aqueous phase was made alkaline (pH 10) with 2M sodium hydroxide and extracted twice with ether. The ether phase was dried ($Na_2SO_4$) and evaporated to yield 250 mg (79%) base, which had a Z/E ratio of 96/4 according to GLC.

Example 7 3-(4-Bromophenyl)-N,N-dimethyl-3-(3-pyridyl)-allylamine

To Z-3-(4-bromophenyl)-3-(3-pyridyl)propenal (288 mg, 1.0 mmol) in 10 ml methanol were added dimethylamine hydrochloride (815 mg, 10 mmol), 1 ml of a 1M solution of sodium hydroxide in methanol, sodium cyanoborohydride (94 mg, 1.5 mmol) and 2 g molecular sieves (3Å). The mixture (pH 8.0) was stirred for 3 hours at room temperature under $N_2$, filtered and the solvent evaporated. The residue was dissolved in 10 ml 2M hydrochloric acid and extracted at pH 4 with ether. The aqueous phase was made alkaline (pH 10) and extracted twice with ether. After drying ($Na_2SO_4$) and evaporation, the residual base (0.24 g, 75%) had a Z/E ratio of 84/16.

Example 8 Z-3-(4-Bromophenyl)-N,N-dimethyl-3-(3-pyridyl)-allylamine

To Z-3-(4-bromophenyl)-3-(3-pyridyl)propenal (288 mg, 1.0 mmol) in 10 ml methanol were added dimethylamine hydrochloride (815 mg, 10 mmol), 0.2 ml of a 1M solution of HCl in ethanol, sodium cyanoborohydride (94 mg, 1.5 mmol) and 2 g molecular sieves (3Å). The mixture (pH 4.5) was stirred for 8 hours at room temperature under $N_2$ and filtered. The filtrate was evaporated, the residue dissolved in 10 ml 2M hydrochloric acid and pH adjusted to 4. After extraction with ether, the aqueous phase was made alkaline (pH 10) with 2 M sodium hydroxide and extracted twice with ether. The ether phase was dried ($Na_2SO_4$) and evaporated to yield 230 mg (72%) base, which had a Z/E ratio of 98/2 according to GLC.

Example 9 Z-3-(4-Bromophenyl)-N,N-dimethyl-3-(3-pyridyl)-allylamine

To Z-3-(4-bromophenyl)-3-(3-pyridyl)propenal (288 mg, 1.0 mmol) in 10 ml methanol were added dimethylamine hydrochloride (815 mg, 10 mmol), sodium cyanoborohydride (94 mg, 1.5 mmol) and 2 g molecular sieves (3Å). The mixture (pH 5.8) was stirred for 5 hours at $0^{o}C$ under $N_2$ and filtered. The filtrate was evaporated, the residue dissolved in 10 ml 2M hydrochloric acid and pH adjusted to 4. After extraction with ether, the aqueous phase was made alkaline (pH 10) with 2M sodium hydroxide and extracted twice with ether. The ether phase was dried ($Na_2SO_4$) and evaporated to yield 260 mg (82%) base, which had a Z/E ratio of 97/3 according to GLC.

Example 10 Z-3-(4-Bromophenyl)-N,N-dimethyl-3-(3-pyridyl)-allylamine

To Z-3-(4-bromophenyl)-3-(3-pyridyl)propenal (288 mg, 1.0 mmol) in 10 ml methanol were added dimethylamine hydrochloride (815 mg, 10 mmol), sodium cyanoborohydride (94 mg, 1.5 mmol) and 2 g molecular sieves (3Å). The mixture (pH 5.8) was stirred for 3 hours at $50^{o}C$ under $N_2$ and filtered. The filtrate was evaporated, the residue dissolved in 10 ml 2M hydrochloric acid and pH adjusted to 4. After extraction with ether, the aqueous phase was made alkaline (pH 10) with 2M sodium hydroxide and extracted twice with ether. The ether phase was dried ($Na_2SO_4$) and evaporated to yield 230 mg (72%) base, which had a Z/E ratio of 97/3 according to GLC.

Best mode of carrying out the invention

Example 3 respresents the best mode of carrying out the invention known to the inventors at present.

Table 1. Ultraviolet spectra in 0.1 M hydrochloric acid or ethanol

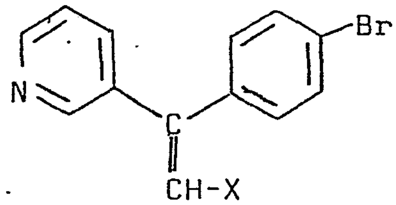

| Example No. | Diastereomer | X | Solvent | Max (nm) | $\varepsilon_{Max}$ | $\lambda_{Min}$ (nm) | $\varepsilon_{Min}$ |
|---|---|---|---|---|---|---|---|
| (A) | E | $CH_2NMe_2$ | HCl | 219<br>237[a)] | 21900<br>18100 | | |
| A | E | CHO | HCl | 226<br>248[a)] | 18300<br>15600 | 218 | 18100 |
| A | E | CHO | EtOH | 227<br>289 | 15900<br>13200 | 255 | 12600 |
| C | Z | $CH_2OH$ | HCl | 248 | 19700 | 224 | 11600 |
| D | Z | CHO | HCl | 312<br>236 | 16400<br>13000 | 273<br>218 | 9500<br>11200 |
| D | Z | CHO | EtOH | 302<br>230 | 22000<br>18100 | 253<br>221 | 11500<br>17700 |
| 1 | E | $CH_2NHMe$ | HCl | 220<br>236[a)] | 20800<br>18800 | | |
| 2 | Z | $CH_2NMe_2$ | HCl | 250 | 19700 | 225 | 14000 |
| 4 | Z | $CH_2NHMe$ | HCl | 248 | 19200 | 224 | 12500 |

a) Shoulder

CLAIMS

1. A novel process for the preparation of a compound of the formula

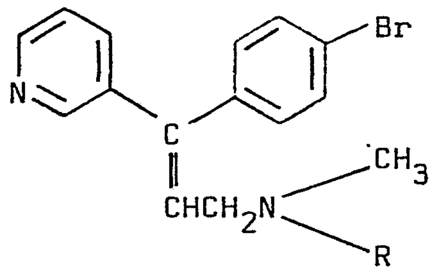

I

or a pharmaceutically acceptable salt thereof, in which formula R is H or $CH_3$ characterized by reductive amination of an aldehyde of the formula

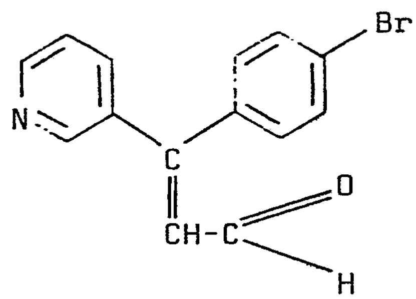

II

with methylamine or dimethylamine in the presence of a reducing agent, to the formation of a compound of formula I, which if desired is converted to a pharmaceutically acceptable salt thereof and/or isolated as an E or Z isomer thereof.

2. A process according to claim 1 characterized in employing as reducing agent a compound selected from lithium cyanoborohydride, sodium cyanoborohydride, sodium borohydride, formic acid, a formamide or an alcoholic potassium hydroxide.

3. A process according to claim 1 or 2 characterized in being carried out under non-oxygen gas atmosphere at a temperature between -20°C and reflux temperature of the solvent used.

4. A process according to claim 2 or 3 characterized in employing sodium cyanoborohydride in an alcoholic solution as the reducing agent, the reaction mixture having a pH between 4 and 8.

5. A process according to claim 4 characterized in employing a pure Z or E isomer of the aldehyde.

6. A process according to one or more of claims 3 to 5 characterized in being carried out at a temperature between 0 and 50°C.

7. The use of a compound of the formula

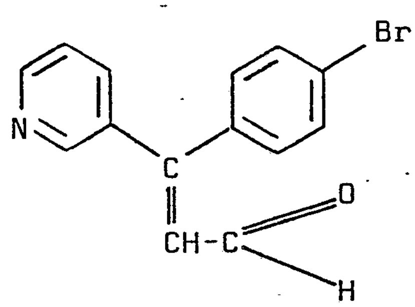

II

as starting material or intermediate for preparation of a therapeutically active compound of the formula

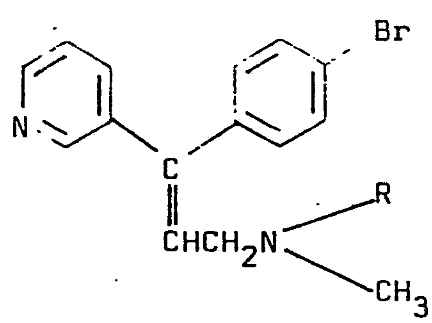

I

or a pharmaceutically acceptable salt thereof, wherein R is H or $CH_3$.

0052588

European Patent Office

# EUROPEAN SEARCH REPORT

Application number
EP 81 85 0205

| Category | Citation of document with indication, where appropriate, of relevant passages | Relevant to claim | CLASSIFICATION OF THE APPLICATION (Int. Cl. 3) |
|---|---|---|---|
| | CH - A - 334 837 (FARBWERKE HOECHST AG) * The whole document * | 1 | C 07 D 213/38<br>A 61 K 31/44//<br>C 07 D 213/48<br>213/30 |
| | -- | | |
| | CHEMICAL ABSTRACTS, vol. 91, 1979, abstract 90641r Columbus, Ohio, US H.S. KIM et al.:"Reductive amination of α,β-unsaturated carbonyl compounds with tetracarbonylhydridoferrate as a reducing agent" & Taehan Hwahakhoe Chi, 1979, 23(2), 99-103 * Abstract * | 1 | TECHNICAL FIELDS SEARCHED (Int.Cl. 3)<br>C 07 D 213/38 |
| | -- | | |
| | US - A - 3 873 621 (M.M. KREEVOY et al.) * The whole document * | 1,2 | |
| | -- | | |
| | Comptes Rendus Hebdomadaires des Séances de l'Académie des Sciences, t. 287 30-10-1978 Série C - 471 Paris, FR J. BASTIDE et al.:"Addition d'acides aminés sur des aldéhydes en présence de cyanoborohydrure de sodium" pages 471-4 * The whole article * | 1 | CATEGORY OF CITED DOCUMENTS<br>X: particularly relevant if taken alone<br>Y: particularly relevant if combined with another document of the same category<br>A: technological background<br>O: non-written disclosure<br>P: intermediate document<br>T: theory or principle underlying the invention<br>E: earlier patent document, but published on, or after the filing date<br>D: document cited in the application<br>L: document cited for other reasons |
| | -- | | |
| | US - A - 2 963 486 (CIBA PHARMACEUTICAL PRODUCTS) * Columns 1-3 * | 1 | |
| | -- ./. | | |
| X | The present search report has been drawn up for all claims | | &: member of the same patent family, corresponding document |

| Place of search | Date of completion of the search | Examiner |
|---|---|---|
| The Hague | 03-02-1982 | VAN BIJLEN |

EPO Form 1503.1 06.78

European Patent Office

**EUROPEAN SEARCH REPORT**

Application number

EP 81 85 0205
-2-

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| | AT - B - 355 574 (PHARMASTRA AG)<br>* Claims *<br>-- | 1 | |
| D | BE - A - 835 802 (ASTRA LAKEMEDEL AB)<br>* Claims *<br>-- | 1 | |
| P | ARZNEIMITTEL FORSCHUNG - DRUG RESERACH 31, No. 3, 1981 Aulendorf, DE<br>J. LUNDSTROM et al.: "Metabolism of Zimelidine in Rat, Dog and Man", pages 486-94<br>* Pages 487, 488, compound 6 *<br>-- | 7 | TECHNICAL FIELDS SEARCHED (Int. Cl.³) |
| E | EP - A - 0 029 420 (ASTRA LAKEMEDEL AB)<br>* Process G; examples 13-14 *<br>---- | 1-6 | |